**Europäisches Patentamt**

**European Patent Office**

**Office européen des brevets**

(19)

(11) **EP 0 828 164 B1**

(12) **EUROPÄISCHE PATENTSCHRIFT**

(45) Veröffentlichungstag und Bekanntmachung des Hinweises auf die Patenterteilung:
**14.08.2002 Patentblatt 2002/33**

(51) Int Cl.7: **G01S 15/58**, A61B 8/06, G01S 15/89

(21) Anmeldenummer: **97890175.9**

(22) Anmeldetag: **04.09.1997**

(54) **Verfahren und Vorrichtung zur Bestimmung der wahren durchschnittlichen Spitzengeschwindigkeit**

Method and apparatus for determining the true average peak velocity

Procédé et dispositif pour déterminer la vrai vitesse de crête moyenne

(84) Benannte Vertragsstaaten:
**AT CH DE FR GB IT LI NL SE**

(30) Priorität: **06.09.1996 AT 158596**

(43) Veröffentlichungstag der Anmeldung:
**11.03.1998 Patentblatt 1998/11**

(73) Patentinhaber: **CARDIOMETRICS, INC.**
**Mountain View California 94043 (US)**

(72) Erfinder: **Jenni, Rolf, Univ.-Prof.Dr.med., Univ., Dep. für 8091 Zürich (CH)**

(74) Vertreter: **Ottevangers, Sietse Ulbe et al Vereenigde, Postbus 87930 2508 DH Den Haag (NL)**

(56) Entgegenhaltungen:
EP-A- 0 286 359     EP-A- 0 573 249
US-A- 5 271 404     US-A- 5 394 876

**Beschreibung**

Die Erfindung betrifft ein Verfahren zur Bestimmung der momentanen durchschnittlichen Spitzengeschwindigkeit (APV) von mit Teilchen beladenen Flüssigkeitsströmen in einem Rohr, unter Verwendung eines in das Rohr eingeführten Doppler-Ultraschall-Transducers, wobei die durchschnittliche Spitzengeschwindigkeit aus einem momentanen Frequenzspektrums ermittelt wird.

Bekannte Apparaturen wurden bereits dazu herangezogen, ein Signal zur Bildung eines Spektrogramms zu liefern, welches die Form einer zweidimensionalen Grauskalendarstellung hat, wobei die Abszisse die Zeit und die Ordinate die Frequenz darstellen und wobei der Grauton eines Punktes die Amplitude der korrespondierenden Frequenzkomponente zum gegebenen Zeitpunkt darstellt. Um eine momentane Spektralspitzenfrequenz - Umhüllende für das Spektrogramm zu bilden, war es nötig, daß eine Fachkraft das Spektrogramm interpretiert und die Grenzen manuell einzeichnet, die durch die höchste Frequenz zu jedem Zeitpunkt definiert ist, die eine signifikante Amplitude über dem Hintergrund-Rauschpegel aufweist. Dieser Vorgang war langwierig und stets etwas subjektiv. Infolgedessen stellte sich die Aufgabe, ein Verfahren und eine Apparatur zu schaffen, die diese umhüllende Linie ohne manuelle Tätigkeit bildet.

Es sind mehrere Vorrichtungen bekannt geworden, die computerisierte Berechnungen zur Bestimmung momentaner Spektralspitzenfrequenzhüllen eines Spektrogramms vornehmen. Jedoch traten dabei viele Probleme und Nachteile auf. Ein Problem liegt darin, daß die Berechnungen sehr kompliziert waren und daher zu langsam erfolgten, um eine Realzeitberechnung der Umhüllenden zu liefern. Ein anderes Problem lag darin, daß die Berechnungen gegenüber der Spektralverteilung oder Amplitude empfindlich waren. Ein weiteres Problem lag darin, daß die Vorrichtungen empfindlich gegenüber Interferenzsignalen waren, die im Spektrogramm vorhanden sein können. Auch die Empfindlichkeit gegenüber zeitlich veränderlichen Rauschpegeln ist aufgetreten.

Diese Nachteile wurden bei einer Vorrichtung und einem Meßverfahren gemäß US-PS 5 271 404 (Cardiometrics Inc.) vermieden, die nach einem darin beschriebenen Verfahren arbeiten.

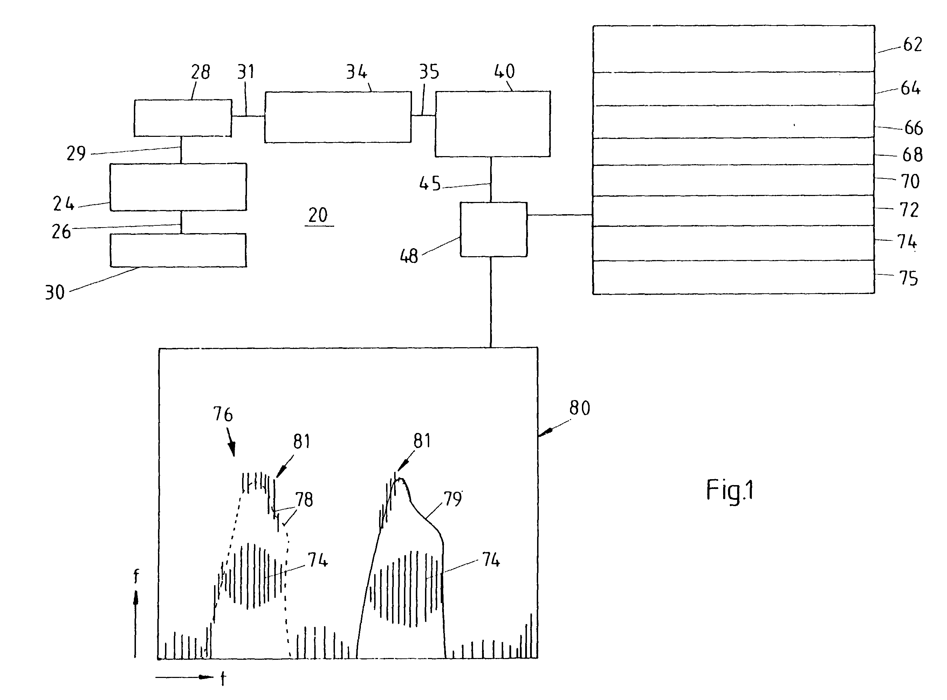

Die Figur 1 zeigt als Blockschaltbild eine Vorrichtung zur Durchführung dieses Verfahrens. Sie umfaßt eine Anzeigevorrichtung mit der Darstellung eines Spektrogramms und der Umhüllenden, die nach dem beschriebenen Verfahren gebildet wird. Die Flüssigkeit ist im folgenden beispielsweise Blut mit Blutkörperchen als Teilchen, und als Röhre dient ein Blutgefäß.

Die Apparatur 20, die die gegenständliche Erfindung umfaßt, wird dazu verwendet, die Dopplerverschiebung von Ultraschallsignalen zu messen und zu verarbeiten, die durch bewegte Blutzellen innerhalb eines Blutgefäßes reflektiert werden. Dadurch wird ein Spektrum erzeugt, aus dem nachher die Umhüllende berechnet wird. Der Apparat umfaßt ein Meßinstrument 24, welches ein mit der Zeit variables Signal erzeugt, wie z.B. ein Führungsdraht mit einem daran montierten Doppler-Ultraschall-Transducer für die Messung der Blutflußgeschwindigkeit in einem menschlichen Blutgefäß, wie z.B. geoffenbart in US-PS 4 967 753 (Cardiometrics Inc.). Unter der Annahme, daß die Vorrichtung 24 ein Doppler-Ultraschall-Transducer ist, umfaßt die Apparatur 20 weiters einen Transmitter 30, der zu gegebenen Wiederholungszeiten über die Leitung 26 zur Vorrichtung 24 RF Impulse schickt, wodurch Ultraschallsignale im Blutstrom erzeugt werden. Die Vorrichtung 24 empfängt die an den Teilchen des Flüssigkeitsstromes reflektierten Ultraschallsignale und ein Empfänger 28 empfängt die erzeugten RF-Signale über die Leitung 29. Die vom Empfänger 28 empfangenen Signale variieren hinsichtlich Frequenz und Amplitude mit der Zeit. Bevorzugt wird das Ultraschallsignal des Dopplertransducers in Vorwärtsrichtung abgegeben, um einen relativ breit gestreuten Ultraschallstrahl zu erzeugen, der den größten Teil des Gefäßquerschnittes abdeckt. Eine "3 dB (one way)" Strahlbreite von 20 bis 90° ist bevorzugt. Solch ein breiter Strahl, der den gesamten oder größten Teil des Gefäßquerschnittes abdeckt, soll bewirken, daß die im Doppler-Spektrum gemessene Spitzenfrequenz weitgehend der höchsten Flußgeschwindigkeit innerhalb des Rohres (Blutgefäß) vor dem Ultraschall-Transducer entspricht, und zwar unabhängig von der genauen Orientierung des Transducers.

Das zeitvariable Signal der Leitung 31 wird einem Meßdaten-Speicher 34 zugeleitet. Von dort werden die gesammelten Signale einem Fourier-Transformator 40 zugeleitet, der bevorzugterweise ein dafür geeigneter Digitalsignalrechner ist. Ein kurzes Segment des gesammelten zeitlich variablen Signals wird über die Leitung 35 einer Fourier-Transformation unterworfen, um ein Frequenzspektrum dieses Segmentes des gesammelten zeitlich variablen Signals zu liefern. Kontinuierlich wird eine Aufeinanderfolge von Spektren erzeugt, indem aufeinanderfolgend kurze Segmente des erhaltenen zeitlich variablen Signals über die Leitung 35 der Fourier-Transformation unterworfen werden.

Das Frequenzspektrum kann in einer passenden Rate wie z.B. 100 Spektrallinien/Sekunde erzeugt werden. Beispielsweise umfaßt ein Frequenzspektrum 256 Frequenzwerte (entspricht dem Y-Wert im Spektrogramm). Jeder Frequenzwert enthält einen Gesamtwert, der der Amplitude der entsprechenden Frequenzkomponente des zugehörigen Segmentes des zeitvariablen Signals auf der Leitung 35 entspricht.

Das von der Vorrichtung 40 erzeugte Spektrum wird der CPU 48 zugeleitet, um die Spektraldaten zu verarbeiten. Die CPU 48 kann eine allgemein übliche CPU sein, wie z.B. ein Intel 80286. Die CPU 48 arbeitet mit einem Befehlssatz für die Verarbeitung der Aufeinanderfolge von Spektren der Leitung 45 und verfügt über einen Speicher bei dem es sich um eine Speicherkarte, ROM, RAM, Diskette oder Kombinationen dieser Speichermedien handeln kann.

In der Folge wird jedes Spektrum so verarbeitet, daß der Spitzenfrequenzwert identifiziert wird, der eine signifikante Amplitude über dem Hintergrund-Rauschpegel aufweist. Die Schwierigkeit bei der Identifizierung der Spitzenfrequenz resultiert aus einer Anzahl von Faktoren wie z.B.: der zeitlich variable Hintergrund-Rauschpegel, elektrische Interferenzsignale und vorübergehende Geräuschspitzen. Weiters muß innerhalb des Spektrums die Spitzenfrequenz innerhalb der Zeitspanne bestimmt werden, bis das nächste Spektrum ermittelt wird, sodaß eine momentane Spitzenfrequenz-Umhüllende erzielt werden kann.

Die Umhüllende 79 und das Geschwindigkeitsspektrogramm 76 kann mittels einer Kathodenstrahlröhre 80 dargestellt werden. Jede vertikale Linie des Geschwindigkeitsspektrogramms entspricht einem einzelnen Spektrum, welches wiederum einem kurzen Segment des zeitlich variablen Signals entspricht. Die Horizontalachse des Geschwindigkeitsspektrogramms repräsentiert die Zeit, während die vertikale Achse die Geschwindigkeit (oder in äquivalenter Weise die Frequenz) wiedergibt. Eine Grauskala kann herangezogen werden, um die spektrale Stärke (Graustufe) jeder Geschwindigkeitskomponente zu jedem Zeitpunkt anzuzeigen. Somit zeigt die Figur 1 eine dünklere Schattierung bei den Mittelfrequenzen jedes Spektrums (74).

Die momentanen durchschnittlichen Spektralspitzengeschwindigkeiten (APV) für eine Aufeinanderfolge von Spektren werden in der Anzeige des Geschwindigkeitsspektrogramms 76 durch eine Reihe von Punkten 78 angezeigt. Zu beachten ist, daß die Spektralwerte oberhalb des Hintergrund-Rauschpegels regelmäßig bei höheren Geschwindigkeiten aufscheinen (bei 81), als die momentanen durchschnittlichen Spektralspitzengeschwindigkeiten, die dem Blutfluß zugeordnet werden können und die als Punkte dargestellt sind. Bei Verfahren, die nicht der vorliegenden Erfindung entsprechen, stören die Spektralwerte oberhalb der durchschnittlichen Spitzengeschwindigkeit den Spitzengeschwindigkeitswert. Die Punkte der Geschwindigkeitsspitzen können unter Bildung einer Umhüllenden 79 miteinander verbunden werden, wodurch die durchschnittlichen momentane Spektralspitzengeschwindigkeits-Wellenform entsteht. Während das Geschwindigkeitsspektrogramm 76 tatsächlich in Frequenzwerten ausgedrückt ist, kann jeder Frequenzwert leicht durch eine Konversion in einen entsprechenden Geschwindigkeitswert umgerechnet werden.

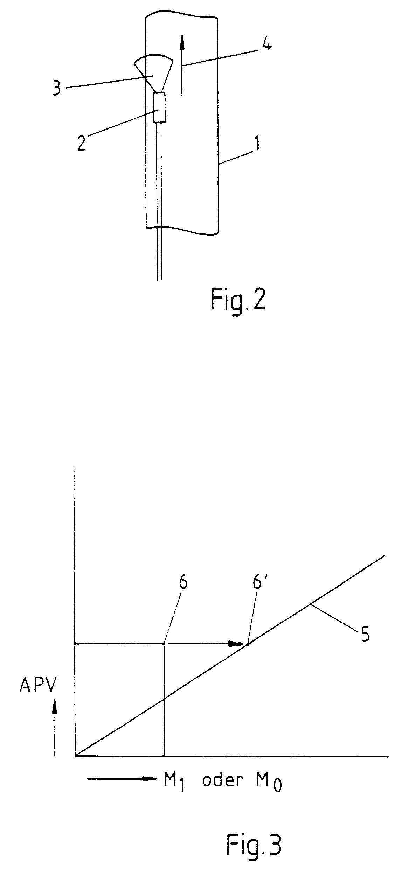

Der vorliegenden Erfindung liegt das Problem zugrunde, daß bei eingeführtem Doppler-Ultraschall-Transducer als Sonde nicht sicher gesagt werden kann, ob die Sonde so im Rohr liegt, daß vom Ultraschall-Strahl tatsächlich die relevanten maximalen Geschwindigkeiten der im Flüssigkeitsstrom schwebenden Teilchen (Blutkörperchen) erfaßt werden. Wie der Fig.2 zu entnehmen ist, kann in der Röhre 1 der Doppler-Ultraschall-Transducer 2 so liegen, daß der gemessene Bereich 3 außerhalb der maximalen Geschwindigkeit 4 der Teilchen liegt, sodaß ein falscher Wert für die APV angezeigt wird.

Gewünscht ist somit ein Indikator dafür, ob die Sonde den richtigen Wert anzeigt, bzw. im Rohr richtig liegt.

Das erfindungsgemäße Verfahren is dadurch gekennzeichnet, daß das Verfahren folgende Schritte umfaßt: das Ermitteln des 0-ten oder 1-sten Spektralmoments des mit dem Frequenzspektrums korrespondierenden Leistungsspektrums; das Messen der Korrelation zwischen dem 0-ten oder 1-sten Spektralmoment und der momentanen durchschnittlichen Spitzengeschwindigkeit; das Vergleichen der gemessenen Korrelation mit einem zugehörigen Standardwert auf einer Geraden (5), welche Gerade der Korrelation zwischen dem 0-te oder 1-ste Spektralmoment und der momentanen durchschnittlichen Spitzengeschwindigkeit bei optimaler Positionierung des Doppler-Ultraschall-Transducers entspricht; und das Bringen des in das Rohr eingeführten Doppler-Ultraschall-Transducers in eine solche Lage, daß die gemessene Korrelation mit dem zugehörigen Standardwert übereinstimmt.

Überraschend hat sich herausgestellt, daß das 0-te und 1-ste Spektralmoment (M0, bzw. M1) dazu herangezogen werden kann, einem Positionsindikator abzugeben. Dabei sind M0 und M1 wie folgt definiert:

$$M0 = \int p(f)\mathrm{d}f$$

$$M1 = \int f.p(f)\mathrm{d}f$$

wobei $p(f)$ das Leistungsspektrum und $f$ die Frequenz des Dopplersignals bezeichnet.

Die erfindungsgemäße Vorrichtung ist dadurch gekennzeichnet, daß die Vorrichtung einen Doppler-Ultraschall-Transducer und eine Anzeigevorrichtung umfaßt, welche Anzeigevorrichtung zur Anzeige der Differenz zwischen der gemessenen Korrelation und einem Standardwert dient, wobei der Standardwert der Korrelation bei optimaler Positionierung des Doppler-Ultraschall-Transducers entspricht.

Nach einem weiteren Merkmal ist die Vorrichtung dadurch gekennzeichnet, daß ein Anzeigeschirm vorgesehen ist, der den Standardwert als Gerade darstellt und den gemessen Wert anzeigt.

Fourier-Analyse wird das Dopplersignal in Frequenzkomponenten aufgeteilt, wobei die Stärke jeder Komponente proportional der Anzahl der reflektierenden Teilchen ist, die mit der entsprechenden Geschwindigkeit strömen. In dem Spektrogramm der Fig.1 entspricht dies dem Grauwert des Spektrums. Wenn der Strahl des Doppler-Ultraschall-Transducers den gesamten Querschnitt des Rohres überdeckt, entspricht das Doppler-Leistungsspektrum der Geschwindigkeitsverteilung und die wahre durchschnittliche Spitzengeschwindigkeit wird gemessen.

Wenn jedoch - was bei Röhren mit größeren Querschnitten der Fall ist - der Ultraschall-Strahl nicht den gesamten Rohrquerschnitt abdeckt, zeigt das Leistungsspektrum nur die Verteilung der Geschwindigkeiten innerhalb dieses beschränkten Flußvolumens. Wenn sich der am raschesten bewegende Teil des Stromes in dem Rohr sich nicht innerhalb dieses Volumens befindet, kommt es zu einer Fehlmessung.

Gemäß vorliegender Erfindung wird aus dem Leistungsspektrum das 0-te oder 1-ste Moment ermittelt und dieser Wert wird mit einem Standardwert verglichen, der geräteabhängig ist. Wenn der gemessene Wert nicht mit dem Standardwert übereinstimmt, liegt eine Fehlmessung vor und der Transducer muß in der Röhre so lange bewegt werden, bis die Werte übereinstimmen.

In Fig.3 ist in einem Diagramm, welches in der Ordinate das APV und in der Abszisse M1 oder M0 aufgetragen hat, die Standardgerade 5 eingezeichnet. Die Lage der Geraden 5 ist von den Meßbedingungen abhängig und wird durch Versuche ermittelt. Diese Gerade enthält die Werte für die optimale Positionierung des Kopfes unabhängig vom Gefäßquerschnitt. Weiters ist ein gemessener Wert 6 eingetragen, der weitab der Geraden 5 liegt und somit anzeigt, daß der gemessene Wert nicht der richtige sein kann. Somit ist es erforderlich, den Doppler-Ultraschall-Transducer 2 mechanisch zu bewegen, bis der gemessene Wert annähernd die optimale Positionierung an der Geraden 5 anzeigt.

Wesentlich ist, daß das Verhältnis zwischen APV und M1 oder MO bei optimaler Positionierung keine Abhängigkeit vom Rohrdurchmesser zeigt. Bei dem für die Versuche verwendenten System FloWire der Firma Cardiometrics Inc. wurde davon ausgegangen, daß der Rohrdurchmesser 6 mm nicht überschreitet.

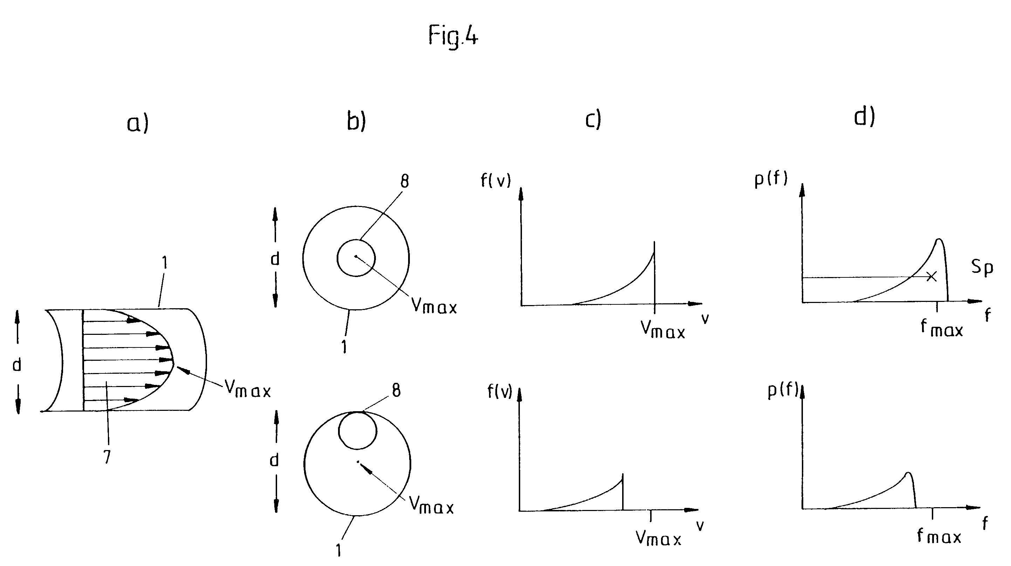

Anhand der Fig. 4 wird die Erfindung nochmals erläutert. Fig. 4a zeigt das Rohr 1 mit dem Durchmesser d im Längsquerschnitt mit dem Strömungsgeschwindigkeitsprofil 7. Vmax liegt in der Rohrmitte. Fig. 4b zeigt das Rohr 1 im Querschnitt, wobei der Strahlkegel 8 des Doppler-Ultraschall-Transducers einmal Vmax umfaßt und in der unteren Darstellung nicht umfaßt. Fig. 4c und 4d zeigen daneben jeweils die zugehörigen Diagramme für die Geschwindigkeitsverteilung f(v) und das Leistungsspektrum p(f). Die gewünschte Positionierung gemäß oberer Reihe der Darstellungen ergibt unter d) eine Kurve, die fmax beinhaltet. Das Moment M1 entspricht dem eingezeichneten Schwerpunkt Sp der unter der Kurve liegenden Fläche. Bei der unterhalb gezeigten Darstellung läge dieser Punkt an einer anderen Stelle, entfernt von der Geraden 5 in dem Diagramm nach Fig. 3.

Bei Testversuchen wurde eine mit Teilchen beladene Flüssigkeit (Blut) aus einem Reservoir mittels einer Quetschpumpe durch vier serielle gerade Silikonrohre mit verschiedenen bekannten Querschnitten gepumpt. In die Rohre wurde ein Doppler-Ultraschall-Transducer eingeführt und dessen Lage jeweils verändert, wobei mit der Apparatur nach Fig. 1 gemessen wurde. Die Flußraten lagen zwischen 18 und 380 ml/min. Die optimale Position der Sonde war jeweils jene mit der maximalen durchschnittlichen Spitzengeschwindigkeit APV und erstem Moment M1 bei gegebener Flußrate. Bei optimaler Position lag die APV zwischen 17 und 83 cm/sec. bei allen vier verschiedenen Rohren, während die entsprechenden Werte für M1 zwischen 19 und 135 Einheiten lag. Die Korrelation zwischen APV und M1 war bei optimaler Positionierung der Sonde praktisch gleich und betrug APV = 12,1 + 0,54 M1, unabhängig vom Rohrdurchmesser. Die Gerade 5 in Fig. 3 entspricht dieser Korrelation.

**Patentansprüche**

1. Verfahren zur Bestimmung der momentanen durchschnittlichen Spitzengeschwindigkeit von mit Teilchen beladenen Flüssigkeitsströmen in einem Rohr, unter Verwendung eines in das Rohr (1) eingeführten Doppler-Ultraschall-Transducers (2), wobei die durchschnittliche Spitzengeschwindigkeit aus einem momentanen Frequenzspektrum ermittelt wird, **dadurch gekennzeichnet, daß** das Verfahren folgende Schritte umfaßt:

   - das Ermitteln des 0-ten oder 1-sten Spektralmoments des mit dem Frequenzspektrum korrespondierenden Leistungsspektrums;
   - das Messen der Korrelation zwischen dem 0-ten oder 1-sten Spektralmoment und der momentanen durchschnittlichen Spitzengeschwindigkeit ;
   - das Vergleichen der gemessenen Korrelation mit einem zugehörigen Standardwert auf einer Geraden (5), welche Gerade der Korrelation zwischen dem 0-ten oder 1-sten Spektralmoment und der momentanen durchschnittlichen Spitzengeschwindigkeit bei optimaler Positionierung des Doppler-Ultraschall-Transducers entspricht; und
   - das Bringen des in das Rohr eingeführten Doppler-Ultraschall-Transducers (2) in eine solche Lage, daß die gemessene Korrelation mit dem zugehörigen Standardwert (5) übereinstimmt.

2. Vorrichtung zur Durchführung des Verfahrens nach Anspruch 1, **dadurch gekennzeichnet, daß** die Vorrichtung einen Doppler-Ultraschall-Transducer (2) und eine Anzeigevorrichtung (80) umfaßt, welche Anzeigevorrichtung zur Anzeige der Differenz zwischen der gemessenen Korrelation und einem Standardwert (5) dient, wobei der Standardwert der Korrelation bei optimaler Positionierung des Doppler-Ultraschall-Transducers entspricht.

3. Vorrichtung nach Anspruch 2, **dadurch gekennzeichnet, daß** ein Anzeigeschirm (80) vorgesehen ist, der den Standardwert als Gerade (5) darstellt und den gemessen Wert (6) anzeigt.

## Claims

1. Method for determining the instantaneous average peak velocity of streams of liquid loaded with particles in a tube, using a Doppler ultrasonic transducer (2) inserted into the tube (1), the average peak velocity being determined from an instantaneous frequency spectrum, **characterised in that** the method comprises the following steps:

   - determining the zero or first spectral moment of the performance spectrum corresponding to the frequency spectrum;

   - measuring the correlation between the zero or first spectral moment and the instantaneous average peak velocity;

   - comparing the measured correlation with an associated standard value on a straight line (5) corresponding to the correlation between the zero or first spectral moment and the instantaneous average peak velocity with optimum positioning of the Doppler ultrasonic transducer; and

   - bringing the Doppler ultrasonic transducer (2) inserted into the tube into a position such that the measured correlation coincides with the associated standard value (5).

2. Apparatus for carrying out the method according to claim 1, **characterised in that** the apparatus comprises a Doppler ultrasonic transducer (2) and a display means (80) displaying the difference between the measured correlation and a standard value (5), the standard value corresponding to the correlation with optimum positioning of the Doppler ultrasonic transducer.

3. Apparatus according to claim 2, **characterised in that** a display screen (80) is provided showing the standard value as a straight line (5) and displaying the measured value (6).

## Revendications

1. Procédé pour déterminer la vitesse de pointe moyenne instantanée d'écoulements liquides chargés de particules dans un tube, moyennant l'utilisation d'un transducteur Doppler à ultrasons (2) inséré dans le tube (1), la vitesse de pointe moyenne étant déterminée à partir d'un spectre instantanée des fréquences, **caractérisé en ce que** le procédé comprend les étapes suivantes:

   - la détermination du moment spectral d'ordre 0 ou du 1-er moment spectral du spectre de puissance correspondant au spectre des fréquences,
   - la mesure de la corrélation entre le moment spectral d'ordre 0 ou le 1-er moment spectral et la vitesse de pointe moyenne instantanée;
   - la comparaison de la corrélation mesurée à une valeur standard associée sur une droite (5), laquelle droite correspond à la corrélation entre le moment spectral d'ordre 0 ou le 1-er moment spectral et la vitesse de pointe moyenne instantanée dans le cas d'un positionnement optimum du transducteur Doppler à ultrasons; et
   - la mise en place du transducteur Doppler à ultrasons (2) introduit dans le tube, dans une position telle que la corrélation mesurée concorde avec la valeur standard associée (5).

2. Dispositif pour la mise en oeuvre du procédé selon la revendication 1, **caractérisé en ce que** le dispositif comprend un transducteur Doppler à ultrasons (2) et un dispositif d'affichage (80), lequel dispositif d'affichage est utilisé pour l'affichage de la différence entre la corrélation mesurée et une valeur standard (5), la valeur standard correspondant à la corrélation dans le cas du positionnement optimum du transducteur Doppler à ultrasons.

3. Dispositif selon la revendication 2, **caractérisé en ce qu'**il est prévu un écran d'affichage (80), qui représente la valeur standard sous la forme d'une droite (5) et affiche la valeur mesurée (6).

Fig.1

Fig. 2

Fig. 3

# Fig.4

a)    b)    c)    d)

EP 0 828 164 B1